(19) Europäisches Patentamt
European Patent Office
Office européen des brevets

(11) EP 0 754 443 A2

# (12) EUROPÄISCHE PATENTANMELDUNG

(43) Veröffentlichungstag: **22.01.1997 Patentblatt 1997/04**

(51) Int. Cl.[6]: **A61K 7/06**

(21) Anmeldenummer: **96104494.8**

(22) Anmeldetag: **21.03.1996**

(84) Benannte Vertragsstaaten: **DE ES FR GB IT**

(30) Priorität: **15.07.1995 DE 19525821**

(71) Anmelder: **Wella Aktiengesellschaft**
**64274 Darmstadt (DE)**

(72) Erfinder: **Racky, Ernst-Dieter**
**65817 Eppstein/Ts. (DE)**

## (54) Haarbehandlungsmittel sowie Verfahren zu dessen Herstellung

(57) Gegenstand der vorliegenden Erfindung ist ein Haarbehandlungsmittel, welches frei von Aniontensiden ist, enthaltend eine Kombination aus

A) 0,5 bis 15 Gewichtsprozent mindestens eines wasserlöslichen kationischen und/oder mindestens eines wasserlöslichen amphoteren Tensids und

B) 0,05 bis 5 Gewichtsprozent Benzoesäure oder deren physiologisch verträglichen Salz oder Saccharin oder dessen physiologisch verträgliches Salz mit einer anorganischen Base oder einer Kombination aus einem physiologisch verträglichen Salz des Saccharins mit einer anorganischen Base und einem physiologisch verträglichen Salz der Benzoesäure,

wobei das Gewichtsverhältnis der Komponente B) zur Komponente A) 2:1 bis 1:10 und der pH-Wert des Mittels 2 bis 7 beträgt sowie ein Verfahren zur Herstellung eines solchen Mittels bei Raumtemperatur.

Das erfindungsgemäße Haarbehandlungsmittel ist bei Raumtemperatur herstellbar, läßt sich problemlos auf eine gewünschte Viskosität einstellen und verbessert die Naß- und Trockenkämmbarkeit des Haares. Zudem bewirkt das Mittel eine Reduzierung der elektrostatischen Aufladung des trockenen Haares, ohne das Haar auch nach mehreren Anwendungszyklen zu belasten.

EP 0 754 443 A2

Printed by Rank Xerox (UK) Business Services
2.13.13/3.4

**Beschreibung**

Gegenstand der Erfindung ist ein aniontensidfreies Haarbehandlungsmittel auf der Basis einer Kombination aus mindestens einem wasserlöslichen kationischen und/oder mindestens einem wasserlöslichem amphoteren Tensid mit Benzoesäure oder Saccharin oder deren Salzen oder mit einer Kombination aus einem Salz des Saccharins und einem Salz der Benzoesäure sowie ein Verfahren zur Herstellung des Mittels bei Raumtemperatur.

Die Haare werden durch Einwirkungen verschiedener Art in ihren physikalischen, chemischen und morphologischen Eigenschaften negativ beeinflußt. So wird durch kosmetische Behandlungen, wie wiederholtes Bleichen, Dauerwellen und Färben, aber auch durch häufiges Waschen der Haare mit entfettenden Tensiden sowie durch Klimaeinflüsse wie Feuchte- und Temperaturunterschiede oder die intensive Einwirkung von Sonnenlicht die Haarstruktur geschädigt. Das Haar wird spröde und verliert seinen Glanz. Das derart geschädigte Haar lädt sich beim Kämmen und Bürsten elektrostatisch auf, und die aufgerauhte Haaroberfläche führt durch die Bildung von Verfilzungen und Verknotungen zu einer schlechten Kämmbarkeit und Entwirrbarkeit des Haares. Haarbehandlungsmittel mit einer pflegenden und kämmbarkeitsverbessernden Wirkung haben daher erhebliche Bedeutung erlangt.

Haarbehandlungsmittel dienen der Verbesserung des Haarzustandes und liegen üblicherweise in Form von Emulsionen vor, die Fettalkohole, Wachse und Öle sowie anionische und kationische Tenside enthalten. Diese Haarbehandlungsmittel in Emulsionsform sind nur mit erheblichem Energie- und Zeitaufwand herstellbar, da die enthaltenden Fette in heißem geschmolzenem Zustand durch intensive Mischer emulgiert werden müssen. Als problematisch hat sich in der Vergangenheit die Einstellung einer anwendungsfreundlichen Viskosität der emulsionsförmigen Haarpflegemittel erwiesen. Insbesondere bei Fettalkoholemulsionen ist die Endviskosität sehr stark vom Kristallisationsverhalten des Fettalkohols während des Abkühlvorgangs abhängig. Unterschiedliche Misch- und Rührgeschwindigkeiten führen zu extremen Viskositätsschwankungen der Masse. Fällt die Dispersion zu dünn an, gibt es keine Möglichkeit mehr, die Konsistenz im kalten Zustand, daß heißt bei Raumtemperatur, zu erhöhen. Man muß die Masse erneut auf Schmelztemperatur erwärmen, um sie dann nochmals unter kontrollierten Bedingungen abzukühlen. Dabei können bestimmte Bestandteile, welche üblicherweise bei niedrigeren Temperaturen zugefügt wurden, zum Beispiel Parfümstoffe, zerstört werden oder sich verflüchtigen.

Bei wenigen emulsionsförmigen Haarbehandlungsmitteln gelingt auch eine Kaltemulgierung. In diesen Fällen werden stets Verdickungsmittel eingesetzt, um die gewünschte Konsistenz zu erreichen.

Wäßrige Gele hingegen sind problemlos bei Raumtemperatur herzustellen. Übliche Haarbehandlungsmittel in Form wäßriger Gele weisen jedoch ein Gehalt an gelbildenden Polymeren auf, die nach mehrfacher Anwendung des gelförmigen Haarbehandlungsmittels auf dem Haar akkumulieren. Der auf dem Haar verbleibende Rückstand der gelbildenden Polymere führt zu einer deutlichen Verschlechterung der Naß- und Trockenkämmbarkeit.

Es bestand daher die Aufgabe, ein bei Raumtemperatur herstellbares Haarbehandlungsmittel zur Verfügung zu stellen, welches die vorstehend genannten Nachteile üblicher emulsionsförmiger oder gelförmiger Haarbehandlungsmittel nicht aufweist.

Es wurde nun gefunden, daß ein Haarbehandlungsmittel, welches frei von Aniontensiden ist und eine Kombination aus

A) 0,5 bis 15 Gewichtsprozent mindestens eines wasserlöslichen kationischen und/oder mindestens eines wasserlöslichen amphoteren Tensids und

B) 0,05 bis 5 Gewichtsprozent Benzoesäure oder deren physiologisch verträgliches Salz oder Saccharin oder dessen physiologisch verträgliches Salz mit einer anorganischen Base oder eine Kombination aus einem physiologisch verträglichen Salz des Saccharins mit einer anorganischen Base und einem physiologisch verträglichen Salz der Benzoesäure

enthält, wobei das Gewichtsverhältnis der Komponente B) zur Komponente A) 2:1 bis 1: 10, bevorzugt 1,5:1 bis 1:5, und der pH-Wert des Mittels 2 bis 7 beträgt, die gestellte Aufgabe in hervorragender Weise erfüllt.

Das erfindungsgemäße Haarbehandlungsmittel ist bei Raumtemperatur, das heißt im üblichen Sprachgebrauch kalt, herstellbar.

Unter Raumtemperatur wird in der vorliegenden Anmeldung ein Temperaturbereich von 15 bis 35°Celsius verstanden, in dem das erfindungsgemäße Mittel ohne Wärmezufuhr herstellbar ist.

Die Einstellung einer bestimmten Viskosität des erfindungsgemäßen Mittel ist problemlos durch die Wahl eines geeigneten Konzentrationsverhältnisses der beiden Komponenten A) und B) im erfindungsgemäßen Bereich möglich. Das erfindungsgemäße gelförmige Haarbehandlungsmittel verbessert die Naß- und Trockenkämmbarkeit des Haares und bewirkt eine Reduzierung der elektrostatischen Aufladung des trockenen Haares, ohne das Haar jedoch auch nach mehreren Anwendungszyklen zu belasten.

Die Viskosität des erfindungsgemäßen Mittels ist von der Gesamtkonzentration der Komponenten A) und B) abhängig. Die Viskosität der erfindungsgemäßen Mittel kann zwischen 1,1 bis 5000 mPas, bevorzugt zwischen 3 und

3000 mPas und besonders bevorzugt zwischen 10 und 2000 mPas, (Millipascalsekunde) bei 25 Grad Celsius und einem Schergefälle von 50 $s^{-1}$ betragen. Bei höheren Konzentrationen an A) und B) kann sich eine Fließgrenze zwischen 0,01 bis 10 Pascal ausbilden, das Mittel zeigt das Fließverhalten eines plastischen Körpers.

Die Viskosität wurde mit einem Haake-Rotationsviskosimeter VT 501, Meßsystem MV-DIN, Schergefälle 50 $s^{-1}$ bei 25 Grad Celsius bestimmt.

Als wasserlösliches Tensid der Komponente A) sind diejenigen kationischen oder amphoteren Tenside geeignet, welche bei einem pH-Wert von 2 bis 7 und einer Konzentration des Tensids von 15 Gewichtsprozent klar wasserlöslich sind und vollständig oder zumindest teilweise in kationischer Form vorliegen.

Das wasserlösliche kationische Tensid der Komponente A) ist bevorzugt ausgewählt aus den $C_{12}$- bis $C_{18}$-Alkylpyridiniumchloriden und -bromiden und den quaternären Ammoniumverbindungen der allgemeinen Formel (I)

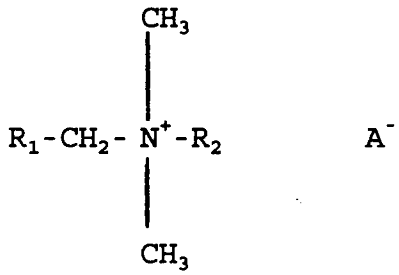

I,

wobei $R_1$ einen $C_7$- bis $C_{15}$-Alkylrest, einen $C_{11}$- bis $C_{17}$-Alkoxycarbonylrest oder einen $C_8$- bis $C_{16}$-Alkylcarboxylmethylrest bedeutet, wenn $R_2$ einen Methylrest darstellt und wobei $R_1$ einen $C_7$- bis $C_{15}$-Alkylrest darstellt, wenn $R_2$ einen Benzyl- oder einen Polyethylenglykol- oder Polypropylenglykolrest, der mit bis zu 5 Ethylenoxid-oder Propylenoxideinheiten alkoxyliert ist, bedeutet und $A^-$ ein Chlorid-, Bromid-, Sulfat-, Hydrogensulfat-, Hydrogenphosphat-, Lactat-, Citrat- oder Methosulfatanion darstellt.

Besonders bevorzugt ist das wasserlösliche kationische Tensid ein Betainester eines $C_{10}$- bis $C_{16}$-Fettalkohols, der allgemeinen Formel (II)

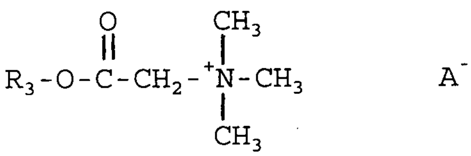

II,

wobei $R_3$ einen $C_{10}$- bis $C_{16}$-Alkylrest und $A^-$ ein Chloridoder Bromid-, Sulfat-, Hydrogensulfat-, Hydrogenphosphat-, Lactat-, Citrat- oder Methosulfatanion darstellt und $R_3$ insbesondere bevorzugt einen Cetyl- oder Laurylrest und $A^-$ bevorzugt ein Chlorid- oder Bromidanion bedeutet.

Die Betainester der $C_{10}$- bis $C_{16}$-Fettalkohole der allgemeinen Formel II können nach der aus der offengelegten japanischen Patentanmeldung 157 750 von 1983 bekannten Synthese hergestellt werden.

Als Komponente A) geeignete kationische Tenside sind beispielsweise Cetyltrimethylammoniumchlorid, das in Form des Handelsproduktes Arquad® 16-25 W von der Firma Akzo Chem. GmbH, Düren, BRD vertrieben wird, Laurylpyridinumchlorid, das beispielsweise von der Firma Henkel KGaA, Düsseldorf, BRD in Form eines Pulvers als Handelsprodukt Dehyquart® C vertrieben wird sowie das Lauryldimethylbenzylammoniumchlorid, welches unter der Handelsbezeichnung Dehyquart® LDB in Form einer 35pro zentigen wäßrigen Lösung von der Henkel KGaA vertrieben wird.

Weiterhin sind als Komponente A) des erfindungsgemäßen Mittels wasserlösliche $C_8$- bis $C_{16}$-Fettsäureester des Cholins geeignet, welche auch als Monoesterquats bezeichnet werden und beispielsweise nach der aus der Tensid Surf. Det. 29 (5), 1992, Seiten 328 bis 332 bekannten Synthese aus Cholinchlorid und Fettsäurechloriden herstellbar sind.

Das amphotere Tensid der Komponente A) ist bevorzugt ausgewählt aus den Carboxylderivaten des Imidazols und den $C_8$- bis $C_{16}$-N-Alkylbetainen.

Als Komponente A) geeignete wasserlösliche amphotere Tenside sind beispielsweise das Lauryldimethylglycin, das die Form einer 15prozentigen wäßrigen Lösung unter der Handelsbezeichnung Rewoteric® AM DL von der Firma Rewo Chem. Werke Steinau, BRD vertrieben wird, Kokosfettalkyldimethylaminoessigsäurebetain (CTFA-Bezeichnung Coco-Betain), das unter der Handelsbezeichnung Dehyton® AB 30 in Form einer 30prozentigen wäßrigen Lösung von

der Firma Henkel KGaA, Düsseldorf, BRD vertrieben wird, das Natriumsalz des 1-(Carboxymethyl)-4,5-dihydro-1-(2-hydroxyethyl)-2-undecyl-1H-imidazolium-hydroxids (CTFA-Bezeichnung: Sodium Lauroamphoacetate), das unter der Handelsbezeichnung Miranol® HM Conc. in Form einer 37prozentigen wäßrigen Lösung von der Firma Miranol Chem. Company Inc., South Brunswick, USA vertrieben wird, das Lauryldimethylglycin (CTFA-Bezeichnung: Laurylbetaine), das unter der Handelsbezeichnung Empigen® BB von der Firma Albright & Wilson Ltd., Whitehaven, Großbritannien und unter der Handelsbezeichnung Armoteric® LB von der Firma Akzo Chemie, Düren, BRD in Form einer 30prozentigen wäßrigen Lösung und von der Firma Rewo Chemische Werke GmbH in Form einer 39prozentigen wäßrigen Lösung vertrieben wird.

Das erfindungsgemäße Mittel enthält bevorzugt 0,5 bis 5 Gewichtsprozent, besonders bevorzugt 0,5 bis 2 Gewichtsprozent, der Komponente A).

Von den als Komponente B) geeigneten Salzen der Benzoesäure sind Natrium-, Kalium-, Magnesium- oder Ammoniumbenzoat bevorzugt.

Von den als Komponente B) geeigneten Salzen des Saccharins mit anorganischen Basen ist das Natriumsaccharinat bevorzugt.

Das erfindungsgemäße Mittel enthält als Komponente B) bevorzugt Natriumbenzoat und/oder Natriumsaccharinat.

Die Komponente B) ist in dem erfindungsgemäßen Mittel vorzugsweise in einer Menge von 0,5 bis 5 Gewichtsprozent enthalten.

Der Wassergehalt des erfindungsgemäßen Haarbehandlungsmittels beträgt vorzugsweise 80 bis 99 Gewichtsprozent.

Der pH-Wert des erfindungsgemäßen Mittels liegt bevorzugt im Bereich von 2 bis 6, besonders bevorzugt zwischen 3 und 6 und kann mit physiologisch verträglichen anorganischen oder organischen Säuren, insbesondere mit Essigsäure, Milchsäure, Zitronensäure, Salzsäure oder Betainhydrochlorid eingestellt werden.

In einer besonderen Ausführungsform kann das erfindungsgemäße Mittel zusätzlich zur Kombination der Komponenten A) und B) 0,01 bis 10 Gewichtsprozent eines wasserlöslichen ein- oder mehrwertigen Alkohols oder eines wasserlöslichen physiologisch verträglichen, einwertigen Metallsalzes einer anorganischen oder organischen Säure als Komponente C) enthalten, wobei der Gewichtsanteil der Komponente C) höchstens die Hälfte der Summe der Gewichtsanteile der Komponenten A) und B) beträgt.

Als für die Komponente C) geeignete Salze sind insbesondere Natriumchlorid, Kaliumchlorid, Calciumchlorid, Natriumsulfat, Magnesiumsulfat, Natriumcitrat, Natriumhydrogensulfat und die Natriumsalze der Phosphorsäure anzuführen.

Die für die Komponente C) geeigneten wasserlöslichen ein- oder mehrwertigen Alkohole sind insbesondere ausgewählt aus niederen Polyglykolen, wie 1,2-Propylenglycol; Isopropanol, Ethanol, Glycerin, Pentaerythrit und Polyglycerinen.

Die Komponente C) kann dem erfindungsgemäßen Mittel zur Feineinstellung einer bestimmten Viskosität zugefügt werden.

In einer besonderen Ausführungsform des erfindungsgemäßen Mittels enthält dieses zur gleichzeitigen Tönung des Haares 0,05 bis 2,0 Gewichtsprozent mindestens eines direkt auf das Haar aufziehenden Haarfarbstoffs, der beispielsweise aus den folgenden Klassen direkt auf das Haar aufziehender Haarfarbstoffe ausgewählt sein kann: aromatische Nitrofarbstoffe, zum Beispiel 2-[(4-Amino-3-nitrophenyl)amino]ethanol (HC Red No. 7), 2-[2-Amino-4-nitrophenyl)amino]ethanol (HC Yellow No. 5), 1,4-Diamino-2-nitrobenzol und 1-Hydroxy-2-aminonitrobenzol, Azofarbstoffe, zum Beispiel Basic Brown 16 (C.I. 12 250), Acid Brown 4 (C.I. 14 805), Aminoketone, wie zum Beispiel 3-[(4-Amino-6-bromo-5oxo-2-naphtalenyl)amino]-N,N,N-trimetylbenzaminiumchlorid (Basic Blue 99, C.I. 56 059), Anthrachinonfarbstoffe, zum Beispiel 1-Amino-4-aminoetylanthrachinon, Disperse Violet 4 (C.I. 61 105) und Triphenylmethanfarbstoffe, zum Beispiel Basic Violet 1 (C. I. 42 535), wobei die Farbstoffe, je nach Art ihrer Substituenten, sauren, nichtionogenen oder basischen Charakter haben können. Die besondere Ausführungsform des erfindungsgemäßen Mittels als Haartönungsmittel weist vorzugsweise eine Viskosität von 13 bis 1390 mPas auf.

In einer weiteren Ausführungsform liegt das erfindungsgemäße Mittel als Dauerwellfixiermittel vor und enthält ein Oxidationsmittel, wie zum Beispiel Hydrogenperoxid, Natrium- und Kaliumbromat, Natriumperborat oder Harnstoffperoxid.

Die Konzentration des Oxidationsmittels ist in Abhängigkeit von der Anwendungszeit (in der Regel etwa 5 bis 15 Minuten) und der Ahwendungstemperatur unterschiedlich; üblicherweise liegt das Oxidationsmittel in einer Konzentration von etwa 0,5 bis 10 Gewichtsprozent vor. Das Dauerwellfixiermittel kann selbstverständlich weitere Stoffe, wie zum Beispiel Netzmittel, schwache Säuren, Puffersubstanzen oder Peroxidstabilisatoren, enthalten.

Das Haarbehandlungsmittel gemäß der vorliegenden Erfindung kann, zusätzlich zu der Kombination aus den Komponenten A) und B), alle diejenigen Bestandteile enthalten, die in Haarbehandlungsmitteln üblicherweise eingesetzt werden, insbesondere nichtionische oberflächenaktive Verbindungen (Tenside), beispielsweise Fettsäurealkanolamide, oxethylierte Sorbitanfettsäureester, in einer Menge von 0,1 bis 10 Gewichtsprozent, vorzugsweise 0,5 bis 2,5 Gewichtsprozent; Pigmente, Parfümöle in einer Menge von etwa 0,5 bis 5,0 Gewichtsprozent; Trübungsmittel, wie zum Beispiel Ethylenglykoldistearat, in einer Menge von etwa 0,5 bis 5,0 Gewichtsprozent; Perlglanzmittel, wie zum Beispiel

ein Gemisch aus Fettsäuremonoalkylolamid und Ethylenglykoldistearat, in einer Menge von etwa 1,0 bis 10,0 Gewichtsprozent; Puffersubstanzen wie beispielsweise Natriumcitrat oder Natriumphosphat, in einer Menge von etwa 0,1 bis 1,0 Gewichtsprozent; Lösungsvermittler, wie zum Beispiel oxethyliertes, gegebenenfalls hydriertes Rizinusöl, in einer Menge von etwa 0,1 bis 1,0 Gewichtsprozent; sowie Farbstoffe, wie zum Beispiel Fluorescein-Natriumsalz, in einer Menge von etwa 0,1 bis 1,0 Gewichtsprozent; weiterhin haarpflegende Zusätze, wie zum Beispiel Fettsäureester, Fettalkohole, kationisch modifizierte natürliche oder kationische synthetische Polymere, wie zum Beispiel kationische Cellulosederivate oder kationische Chitinderivate; Pflegestoffe, wie zum Beispiel Lanolinderivate, Cholesterin und Pantothensäure, in einer Menge von etwa 0,1 bis 10 Gewichtsprozent; außerdem physiologisch verträgliche anorganische Salze, wie zum Beispiel Calciumchlorid oder Magnesiumsulfat sowie ferner Feuchthaltemittel; Pflanzenextrakte; Proteinhydrolysate; Anfärbefarbstoffe; Lichtschutzmittel; Antioxidantien; Komplexbildner; Antischuppenwirkstoffe; kosmetische Öle und Wachse und Konservierungsstoffe.

Das erfindungsgemäße Mittel kann bis zu 0,5 Gewichtsprozent gelbildende natürliche oder synthetische Polymere enthalten, ist jedoch bevorzugt frei von gelbildenden natürlichen oder synthetischen Polymeren. Das erfindungsgemäße Mittel kann in Form einer Suspension, Emulsion oder eines Gels vorliegen und kann die für Haarbehandlungsmittel üblichen Zusatzstoffe bevorzugt in einer Menge von 0,01 bis 20 Gewichtsprozent enthalten.

Das erfindungsgemäße Mittel kann in Form eines mit Hilfe einer geeigneten mechanisch betriebenen Sprühvorrichtung versprühbaren Haarbehandlungsmittels oder eines Non-Aerosol-Schaumes vorliegen.

Unter mechanischen Sprühvorrichtungen sind solche Vorrichtungen zu verstehen, welche das Versprühen einer Flüssigkeit ohne Verwendung eines verflüssigten Treibmittels ermöglichen. Als geeignete mechanische Sprühvorrichtung kann beispielsweise eine Sprühpumpe oder ein mit einem Sprühventil versehener elastischer Behälter, in den das erfindungsgemäße kosmetische Mittel unter Druck abgefüllt wird, wobei sich der elastische Behälter ausdehnt und aus dem sich das Mittel infolge der Kontraktion des elastischen Behälters bei Öffnen des Sprühventils kontinuierlich entnehmen läßt, verwendet werden.

Das erfindungsgemäße Mittel kann auch unter Zusatz eines Treibmittels in einem Druckbehälter abgefüllt werden, wobei das Präparat als Schaum entnommen wird und mittels eines mit einer Auftragdüse versehenen Ventils leicht dosiert und bequem auf dem Haar verteilt werden kann. Als Treibmittel sind beispielsweise niedere Alkane, wie zum Beispiel n-Butan, i-Butan und Propan oder auch Dimethylether sowie ferner bei den in Betracht kommenden Drücken gasförming vorliegende Treibmittel, wie beispielsweise $N_2$, $N_20$ und $CO_2$ sowie Gemische der vorstehend genannten Verbindungen geeignet. Die Treibmittel sind in diesem Mittel zweckmäßigerweise in einer Menge von etwa 2 bis 10 Gewichtsprozent enthalten.

Das erfindungsgemäße Mittel ist bevorzugt ein Haarpflegemittel und kann als Leave-in-Conditioner als Kurschaum, als Haarspülung und als Kurpackung vorliegen.

Erfindungsgemäße Mittel, welche nach der Anwendung ausgespült werden, wie Haarkurmittel und Spülungen, werden üblicherweise nach der Haarwäsche, in dem handtuchtrockenen Haar je nach Haarfülle in einer Menge von etwa 10 bis 30 g verteilt. Nach einer Einwirkungszeit von 1 bis 30 Minuten, vorzugsweise 3 bis 15 Minuten, wird das Haar mit Wasser gespült und sodann getrocknet.

Die Einwirkungszeit des erfindungsgemäßen Mittels hängt innerhalb der gegebenen zeitlichen Grenzen von dem Verwendungszweck des Mittels ab. Handelt es sich um eine Spülung, so beläßt man sie 1 bis 5 Minuten lang auf dem Haar, während bei einer Haarkur eine Einwirkungsdauer von 3 bis 15 Minuten üblich ist und bei einer Haarkur mit gleichzeitiger Tönung des Haares die Einwirkungszeit 10 bis 30 Minuten beträgt.

Bei erfindungsgemäßen Mitteln, die nach der Anwendung nicht wieder ausgespült werden, wie beispielsweise Leave-in-Conditioner, werden je nach Haarfülle 2 bis 4 g des Mittels im handtuchtrockenen Haar verteilt. Sodann wird das Haar getrocknet.

Gegenstand der vorliegenden Anmeldung ist weiterhin ein Verfahren zur Herstellung des erfindungsgemäßen Haarbehandlungsmittels, bei dem bei Raumtemperatur die Komponente A) in 50 bis 90 Prozent der Gesamtwassermenge des Mittels gelöst wird und sodann mit der in 10 bis 50 Prozent der Gesamtwassermenge gelösten Komponente B) bei Raumtemperatur vermengt wird.

Der wäßrigen Lösung der Komponente A) werden vor dem Vermischen mit der Lösung der Komponente B) gegebenenfalls für Haarbehandlungsmittel übliche Zusatzstoffe zugefügt.

Wenn das erfindungsgemäße Mittel in der vorstehend beschriebenen besonderen Ausführungsform zusätzlich zur Kombination der Komponenten A) und B) die Komponente C) enthält, wird die Komponente C) bei der Herstellung des erfindungsgemäßen Mittels nach dem Vermischen der wäßrigen Lösungen der Komponenten A) und B) zugefügt, wobei die wäßrige Lösung der Komponente A) gegebenenfalls für Haarbehandlungsmittel übliche Zusatzstoffe enthalten kann.

Die mit dem erfindungsgemäßen Mittel behandelten Haare zeigen eine hervorragende Naß- und Trockenkämmbarkeit, einen angenehmen Griff und einen ansprechenden Glanz im getrockneten Zustand.

Die nachstehenden Beispiele sollen den Gegenstand der Erfindung näher erläutern.

## Beispiele

### Beispiele 1 bis 5: Haarbehandlungsmittel

| Beispiel | 1 | 2 | 3 | 4 | 5 |
|---|---|---|---|---|---|
| Betainlaurylester | 3,20 g | 3,20 g | 6,40 g | -- | -- |
| Betaincetylester | -- | -- | -- | 3,80 g | 3,80 g |
| Natriumbenzoat | -- | -- | -- | 1,20 g | 1,20 g |
| Natrium-Saccharinat Dihydrat | 1,60 g | 2,00 g | 3,20 g | -- | -- |
| 1,2-Propylenglycol | -- | -- | -- | -- | 1,00 g |
| Zitronensäure-Monohydrat | 0,10 g | 0,10 g | 0,10 g | 0,10 g | 0,10 g |
| Wasser | 95,10 g<br>100,00 g | 94,70 g<br>100,00 g | 90,30 g<br>100,00 g | 94,90 g<br>100,00 g | 94,95 g<br>100,00 g |
| **Beispiel** | **1** | **2** | **3** | **4** | **5** |
| Viskosität [mPas] (25°C / 50 $s^{-1}$) | 16,0 | 19,0 | 73,0 | 640 | 420 |

### Beispiele 6 bis 10: Haarbehandlungsmittel

| Beispiel | 6 | 7 | 8 | 9 | 10 |
|---|---|---|---|---|---|
| Cetyltrimethylammoniumchlorid | 1,60 g | 1,60 g | 2,50 | g 5,00 g | 8,80 g |
| Natriumbenzoat | 0,70 g | 0,70 g | 1,20 | g 2,20 g | 4,10 g |
| Natriumchlorid | -- | 1,00 g | -- | -- | -- |
| Zitronensäure-Monohydrat | 0,05 g | 0,05 g | 0,30 | g 0,30 g | 0,30 g |
| Wasser | 97,65 g<br>100,00 g | 97,65 g<br>100,00 g | 96,00<br>100,00 g | g 92,50 g<br>100,00 g | 86,80 g<br>100,00 g |
| **Beispiel** | **6** | **7** | **8** | **9** | **10** |
| Viskosität [mPas] (25°C / 50 $s^{-1}$) | 3,0 | 4,0 | 15,0 | 550 | 2600 |

### Beispiel 11: Haartönungsmittel

| | |
|---|---|
| 1,25 g | Cetyltrimethylammoniumchlorid |
| 0,50 g | Natriumbenzoat |
| 0,75 g | Natriumsaccharinat |
| 0,01 g | Zitronensäure |
| 0,70 g | 2[(4-Amino-3-nitrophenyl)amino]ethanol |
| 0,25 g | 2[(2-Amino-4-nitrophenyl)amino]ethanol |
| 0,05 g | 3-[(4-Amino-6-bromo-5-oxo-2-naphthalenyl) amino]-N,N,N-trimethylbenzaminium Chlorid (C.I. 56 059) |
| 96,49 g | Wasser |
| 100,00 g | |

Das Haartönungsmittel gemäß Beispiel 11 weist eine Viskosität von 13,3 mPas bei 25 Grad Celsius und einem Schergefälle von 50 $s^{-1}$ auf. Das mit dem Haartönungsmittel gemäß Beispiel 11 behandelte Haar weist einen warmen Rotbraunton auf.

**Beispiel 12: Haartönungsmittel**

| | |
|---|---|
| 10,00 g | Cetyltrimethylammoniumchlorid |
| 2,00 g | Natriumsaccharinat |
| 0,02 g | Zitronensäure |
| 0,70 g | 2[(4-Amino-3-nitrophenyl)amino]ethanol |
| 0,25 g | 2[(2-Amino-4-nitrophenyl)amino]ethanol |
| 0,05 g | 3-[(4-Amino-6-bromo-5-oxo-2-naphthalenyl)amino]-N,N,N-trimethylbenzaminium Chlorid (C.I. 56 059) |
| 86,98 g | Wasser |
| 100,00 g | |

Das Haartönungsmittel gemäß Beispiel 12 weist eine Viskosität von 1390 mPas bei 25 Grad Celsius und einem Schergefälle von $50^{-1}$ auf. Das mit dem Haartönungsmittel gemäß Beispiel 12 getönte Haar weist einen warmen Rotbraunton auf.

**Beispiel 13: Haartönungsmittel**

| | |
|---|---|
| 1,00 g | Cetyltrimethylammoniumchlorid |
| 0,50 g | Natriumbenzoat |
| 0,50 g | Natriumsaccharinat |
| 0,01 g | Zitronensäure |
| 0,15 g | 3[(4-Amino-6-bromo-5-oxo-2-naphthalenyl)-amino]-N,N,N-trimethylbenzaminium Chlorid (C.I. 56 059) |
| 0,03 g | [8-[(p-Aminophenyl)azo]-7-hydroxy-2-naphthyl]-trimethylammonium Chlorid (Basic Brown 16, C.I. 12 250) |
| 0,20 g | 1-Amino-4-aminoethylanthrachinon (C.I. 61 105) |
| 0,15 g | 2,2'-[(4[2-Hydroxyethyl)amino]-3-nitrophenyl]imino]bis-ethanol |
| 88,46 g | Wasser |
| 100,00 g | |

Das Haartönungsmittel gemäß Beispiel 13 weist eine Viskosität von 69,3 mPas bei 25 Grad Celsius und einem Schergefälle von 50 $s^{-1}$ auf und tönt das damit behandelte Haar violettblaugrau.

**Beispiel 14: Haartönungsmittel**

| | |
|---|---|
| 2,50 g | Cetyltrimethylammoniumchlorid |
| 0,50 g | Natriumbenzoat |
| 2,00 g | Natriumsaccharinat |
| 0,02 g | Zitronensäure |
| 0,15 g | 3[4-Amino-6-bromo-5-oxo-2-naphthalenyl)-amino]-N,N,N-trimethylbenzaminium Chlorid (C.I. 56 059) |
| 0,03 g | [8-[(p-Aminophenyl)azo]-7-hydroxy-2-naphthyl]-trimethylammonium Chlorid (Basic Brown 16 C.I. 12 250) |
| 0,20 g | 1-Amino-4-aminoethylanthrachinon (C.I. 61 105) |
| 0,15 g | 2,2'-[[4-[2-Hydroxyethyl)amino]-3-nitrophenyl]imino]bis-ethanol |
| 88,46 g | Wasser |
| 100,00 g | |

Das Haartönungsmittel gemäß Beispiel 14 weist eine Viskosität von 229 mPas bei 25 Grad Celsius und einem Schergefälle von $50 s^{-1}$ auf und tönt das Haar violettblaugrau.

**Vergleichsbeispiele 15 bis 17: Dauerwellfixiermittel**

Die Viskosität des erfindungsgemäßen Beispiels 15 wurde mit Viskositäten der nicht erfindungsgemäßen Beispiele 16 und 17 verglichen.

| **Beispiel** | **15** | **16** | **17** |
|---|---|---|---|
| Lauryldimethylglycin | 4,50 g | 4,50 g | |
| Natriumsalz des 1-(Carboxymethyl)-4,5-dihydro-1-(2-hydroxyethyl)-2-undecyl-1H-imidozoliumhydroxid | 0,50 g | 0,50 g | -- |
| Natriumsaccarinat | 2,00 g | -- | 2,00 g |
| Phenacetin | 0,04 g | 0,04 g | 0,04 g |
| Polydimethyldiallylammoniumchlorid | 0,40 g | 0,40 g | 0,40 g |
| Phosphorsäure, 85prozentig | 0,50 g | 0,50 g | 0,50 g |
| **Beispiel** | **15** | **16** | **17** |
| Wasserstoffperoxid 50prozentige Lösung | 5,00 g | 5,00 g | 5,00 g |
| Ethylendiamintetraessigsäure | 0,20 g | 0,20 g | 0,20 g |
| Wasser | 86,86 g<br>100,00 g | 86,86 g<br>100,00 g | 91,86 g<br>100,00 g |
| pH-Wert | 3,6 | 3,5 | 2,3 |
| Viskosität [mPas], (25°C / 50 $s^{-1}$) | 13,0 | 2 | 2 |

Das erfindungsgemäße Dauerwellfixiermittel gemäß Beispiel 15 ist klar und viskos, während die nicht erfindungsgemäßen Beispiele 16 und 17 dünnflüssig sind und Beispiel 17 sogar eine flockige weiße Dispersion darstellt.

**Beispiele 18 bis 21: Haarbehandlungsmittel**

| Beispiel | 18 | 19 | 20 | 21 |
|---|---|---|---|---|
| Kokosfettalkyldimethylaminoessigsäurebetain | 10,00 g | 10,00 g | 6,00 g | 6,00 g |
| Natriumsaccharinat | 1,67 g | 3,33 g | 1,00 g | 2,00 g |
| Wasser | 88,33 g | 86,67 g | 93,00 g | 92,00 g |
| | 100,00 g | 100,00 g | 100,00 g | 100,00 g |

Der pH-Wert der Beispiele 18 bis 21 wurde mit Salzsäure auf pH = 2,4 umgestellt.

| Beispiel | 18 | 19 | 20 | 21 |
|---|---|---|---|---|
| Viskosität [mPas], 25° C / $50^{-1}$ | 41 | 100 | 12 | 49,6 |

**Beispiele 22 bis 25: Haarbehandlungsmittel**

| Beispiel | 22 | 23 | 24 | 25 |
|---|---|---|---|---|
| Lauryldimethylglycin | 10,00 g | 10,00 g | 10,00 g | 10,00 g |
| Natriumsaccharinat | 2,50 g | 3,00 g | 3,50 g | 5,00 g |
| Wasser | 87,50 g | 87,00 g | 86,50 g | 85,00 g |
| | 100,00 g | 100,00 g | 100,00 g | 100,00 g |

Der pH-Wert der Beispiele 22 bis 25 wurde mit Salzsäure auf pH = 3,9 umgestellt.

| Beispiel | 22 | 23 | 24 | 25 |
|---|---|---|---|---|
| Viskosität [mPas], 25°C / $50s^{-1}$ | 18 | 34 | 50 | 81 |

**Beispiele 26 und 27: klare Kurspülungen**

| Beispiel | 26 | 27 |
|---|---|---|
| Cetyltrimethylammoniumchlorid | 1,00 g | 1,00 g |
| Natriumsaccharinat | 0,75 g | 0,75 g |
| Zitronensäure | 0,20 g | 0,20 g |
| Pflanzenextrakt (Extapone 5 Special® der Firma Dragoco, Holzminden, Deutschland) | 0,20 g | 0,20 g |
| Dipalmitoylethylhydroxyethylmethylammoniummethosulfat | ---- | 0,05 g |
| Acid Yellow 3 (C.I. 47005) | 0,13 g | 0,13 g |
| Pigment Green 7 (C.I. 74260) | 0,07 g | 0,07 g |
| Parfüm | 0,10 g | 0,10 g |
| Wasser | 97,55 g | 97,50 g |
| | 100,00 g | 100,00 g |
| pH-Wert | 2,7 | 2,6 |
| Viskosität [mPas] 25°C / 50 $s^{-1}$ | 197 | 125 |

**Beispiele 28 bis 30: klare Kurspülungen**

| Beispiel | 28 | 29 | 30 |
|---|---|---|---|
| Cetyltrimethylammoniumchlorid | 0,83 g | 0,83 g | 0,83 g |
| Natriumsaccharinat | 0,67 g | 0,67 g | 0,67 g |
| Proteinhydrolysat (Nutrilan® der Firma Henkel, Düsseldorf, Deutschland) | 2,00 g | ---- | ---- |
| Sojaproteinhydrolysat (Croquat Soya® der Firma Croda | ---- | 0,50 g | ---- |
| Kollagenpartialhydrolysat (Gelita-Sol C® der Firma Deutsche Gelatine-Fabriken Stoess & Co. GmbH, Eberbach, Deutschland | ---- | ---- | 1,00 g |
| Milchsäure | 0,07 g | 0,07 g | 0,07 g |
| Acid Yellow 3 (C.I. 47005)<br>Pigment Green 7 (C.I. 74260) | 0,09 g<br>0,05 g | 0,09 g<br>0,05 g | 0,09 g<br>0,05 g |
| Parfüm | 0,10 g | 0,05 g | 0,04 g |
| **Beispiel** | **28** | **29** | **30** |
| Wasser | 96,19 g<br>100,00 g | 97,74 g<br>100,00 g | 97,25 g<br>100,00 g |
| pH-Wert | 4,1 | 3,5 | 4,0 |
| Viskosität [mPas] 25°C / 50 $s^{-1}$ | 90 | 98 | 135 |

**Beispiele 31 und 32: trübe Kurspülungen**

| Beispiel | 31 | 32 |
|---|---|---|
| Cetyltrimethylammoniumchlorid | 0,83 g | 0,83 g |
| Natriumsaccharinat | 0,67 g | 0,67 g |
| Stearylalkohol | 1,00 g | ---- |
| Titan(IV)oxid | ---- | 0,50 g |
| Milchsäure | 0,50 g | 0,50 g |
| Pigment Green 7 (C.I. 74260) | 0,15 g | ---- |
| Acid Yellow 3 (C.I. 47005) | 0,05 g | ---- |
| Parfüm | 0,10 g | 0,05 g |
| Wasser | 96,70 g | 97,45 g |
| | 100,00 g | 100,00 g |
| pH-Wert | 4,1 | 3,2 |
| Viskosität [mPas] 25°C / 50 $s^{-1}$ | 114 | 166 |

**Beispiele 33 und 34: Spülungen**

| Beispiel | 33 | 34 |
|---|---|---|
| Betainlaurylesterchlorid | 0,80 g | 0,80 g |
| Natriumsaccharinat | 0,65 g | 0,65 g |
| Cocosmonoethanolamid | 5,00 g | ---- |
| Cocosdiethanolamid | ---- | 5,00 g |
| Milchsäure | 0,40 g | 0,40 g |
| Pigment Green 7 (C.I. 74260) | 0,15 g | 0,15 g |
| Acid Yellow 3 (C.I. 47005) | 0,05 g | 0,05 g |
| Parfüm | 0,1 g | 0,08 g |
| Wasser | 92,85 g | 92,87 g |
| | 100,00 g | 100,00 g |
| pH-Wert | 4,6 | 4,3 |
| Viskosität [mPas] 25°C / 50 $s^{-1}$ | 113 | 76 |

Alle Prozentangaben dieser Anmeldung stellen, sofern nicht anders angegeben, Gewichtsprozente dar.

**Patentansprüche**

1. Haarbehandlungsmittel, welches frei von Aniontensiden ist, enthaltend eine Kombination aus

   A) 0,5 bis 15 Gewichtsprozent mindestens eines wasserlöslichen kationischen und/oder mindestens eines wasserlöslichen amphoteren Tensids,

   B) 0,05 bis 5 Gewichtsprozent Benzoesäure oder deren physiologisch verträgliches Salz oder Saccharin oder dessen physiologisch verträgliches Salz mit einer anorganischen Base oder einer Kombination aus einem physiologisch verträglichen Salz des Saccharins mit einer anorganischen Base und einem physiologisch verträglichen Salz der Benzoesäure,

   wobei das Gewichtsverhältnis der Komponente B) zur Komponente A) 2:1 bis 1:10 und der pH-Wert des Mittels 2 bis 7 beträgt.

2. Haarbehandlungsmittel nach Anspruch 1, dadurch gekennzeichnet, daß das kationische Tensid ausgewählt ist aus den $C_{12}$- bis $C_{18}$-Alkylpyridiniumchloriden und -bromiden und den quaternären Ammoniumverbindungen der allgemeinen Formel (I)

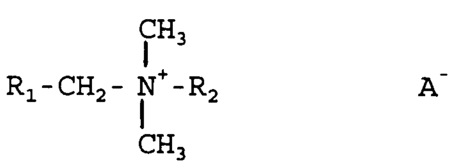

I,

   wobei $R_1$ einen $C_7$- bis $C_{15}$-Alkylrest, einen $C_{11}$- bis $C_{17}$-Alkoxycarbonylrest oder einen $C_8$- bis $C_{16}$-Alkylcarboxylmethylrest bedeutet, wenn $R_2$ einen Methylrest darstellt und wobei $R_1$ einen $C_7$- bis $C_{15}$-Alkylrest darstellt, wenn $R_2$ einen Benzyl- oder einen Polyethylenglykol oder Polypropylenglykolrest, der mit bis zu 5 Ethylenoxid- oder Propylenoxideinheiten alkoxyliert ist, bedeutet und $A^-$ ein Chlorid-, Bromid-, Sulfat-, Hydrogensulfat, Hydrogenphosphat-, Lactat, Citrat- oder Methosulfatanion darstellt.

3. Haarbehandlungsmittel nach Anspruch 1 oder 2, dadurch gekennzeichnet, daß das kationische Tensid ein Betainester eines $C_{10}$- bis $C_{16}$-Fettalkohols der allgemeinen Formel (II)

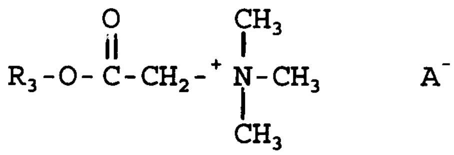

II,

wobei $R_3$ einen $C_{10}$- bis $C_{16}$-Alkylrest und $A^-$ ein Chlorid- oder Bromid-, Sulfat-, Hydrogensulfat-, Hydrogenphosphat-, Lactat, Citrat- oder Methosulfatanion darstellt, ist.

4. Haarbehandlungsmittel nach einem der Ansprüche 1 bis 3, dadurch gekennzeichnet, daß das amphotere Tensid ausgewählt ist aus den $C_8$- bis $C_{16}$-N-Alkylbetainen und den Carboxylderivaten des Imidazols.

5. Haarbehandlungsmittel nach einem der Ansprüche 1 bis 4, dadurch gekennzeichnet, daß es als Komponente C) 0,01 bis 10 Gewichtsprozent eines wasserlöslichen ein- bis fünfwertigen Alkohols oder eines wasserlöslichen, physiologisch verträglichen, einwertigen Metallsalzes einer anorganischen oder organischen Säure enthält, wobei der Gewichtsanteil der Komponente C) höchstens die Hälfte der Summe der Gewichtsanteile der Komponenten A) und B) beträgt.

6. Verfahren zur Herstellung eines Haarbehandlungsmittels nach einem der Ansprüche 1 bis 5, dadurch gekennzeichnet, daß bei Raumtemperatur die Komponente A) in 50 bis 90 Prozent der Gesamtwassermenge des Mittels gelöst wird und sodann mit der in 10 bis 50 Prozent der Gesamtwassermenge gelösten Komponente B) bei Raumtemperatur vermengt wird.

7. Verfahren nach Anspruch 6, dadurch gekennzeichnet, daß der wäßrigen Lösung der Komponente A) vor dem Vermischen mit der Lösung der Komponente B) für Haarbehandlungsmittel übliche Zusatzstoffe zugefügt werden.

8. Verfahren nach einem der Ansprüche 6 oder 7, dadurch gekennzeichnet, daß nach dem Vermischen der wäßrigen Lösungen der Komponenten A) und B) die Komponente C) zugefügt wird.